(19) Europäisches Patentamt
European Patent Office
Office européen des brevets

(11) Veröffentlichungsnummer: **0 222 268 B1**

# (12) EUROPÄISCHE PATENTSCHRIFT

(45) Veröffentlichungstag der Patentschrift: **04.01.89**

(51) Int. Cl.[4]: **C07C 147/06**

(21) Anmeldenummer: **86115066.2**

(22) Anmeldetag: **30.10.86**

## (54) Verfahren zur Herstellung von Aryloxethylsulfonen.

(30) Priorität: **07.11.85 DE 3539477**

(43) Veröffentlichungstag der Anmeldung: **20.05.87 Patentblatt 87/21**

(45) Bekanntmachung des Hinweises auf die Patenterteilung: **04.01.89 Patentblatt 89/1**

(84) Benannte Vertragsstaaten: **CH DE FR GB IT LI**

(56) Entgegenhaltungen:
**EP-A- 0 115 328**
**US-A- 3 092 672**

(73) Patentinhaber: **HOECHST AKTIENGESELLSCHAFT, Postfach 80 03 20, D-6230 Frankfurt am Main 80(DE)**

(72) Erfinder: **Papenfuhs, Theodor, Dr., Heinrich-Bleicher-Strasse 40, D-6000 Frankfurt am Main 50(DE)**
Erfinder: **Koller, Wolfgang, Dr., Im Brühl 14, D-6231 Sulzbach(DE)**
Erfinder: **Kühn, Walter, Dr., Am Sonnenhang 7, D-6238 Hofheim am Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein einstufiges Verfahren zur Herstellung von Aryloxethylsulfonen, welche wichtige Vorprodukte zur Herstellung von Reaktivfarbstoffen der Vinylsulfonreihe darstellen.

Phenyloxethylsulfone der allgemeinen Formel (I)

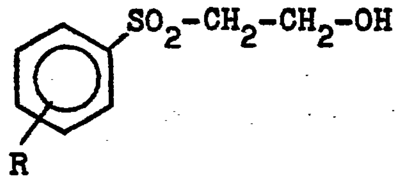

(I)

in welcher R ein Wasserstoffatom oder eine Alkylgruppe bedeutet, werden bisher nach zwei bekannten Verfahren, die jeweils durch mindestens zwei Syntheseschritte gekennzeichnet sind, hergestellt.

Das eine Verfahren besteht darin, daß man Aromaten der Formel (II) sulfochloriert zu den Verbindungen der Formel (III), diese zu den Sulfinsäuren der Formel (IV) reduziert u. letztere oxethyliert zu den Sulfonen der Formel (I) gemäß dem Reaktionsschema

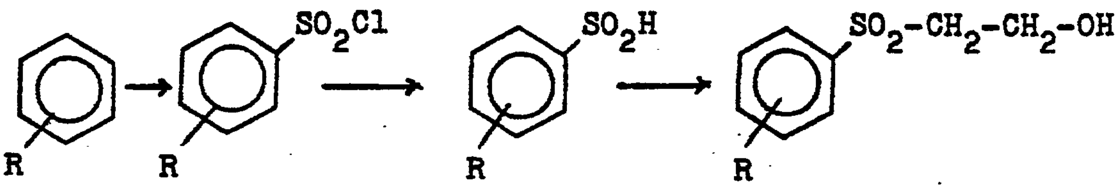

(II) (III) (IV) (I)

(vgl. hierzu beispielsweise EP-OS 0 115 328).

Beim anderen bekannten Verfahren erfolgt ein Austausch von Halogen in aktivierten Aromaten der Formel (V) durch einen Mercaptoethanolrest zu den Sulfiden der Formel (VI) u. deren Oxidation zu den Sulfonen der Formel (I) gemäß dem Reaktionsschema

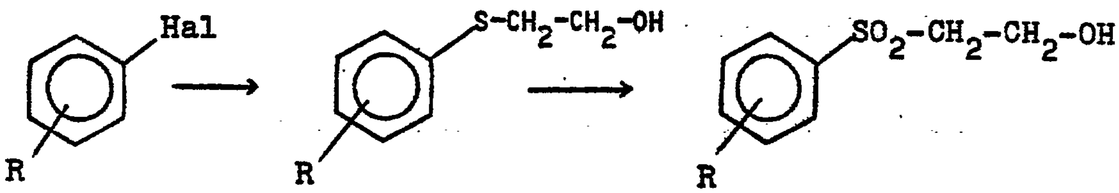

(V) (VI) (I)

(vgl. hierzu beispielsweise DE-OS 3 343 421).

Die beiden vorstehend genannten, bekannten Mehrstufenverfahren zur Herstellung von Aryloxethylsulfonen der Formel (I) werden technisch praktiziert. Einstufenverfahren zur Einführung der Oxethylsulfongruppe sind aber bisher nicht bekannt.

Aus der US-Patentschrift 3 092 672 geht hervor, daß die Umsetzung von Benzol oder alkylsubstituierten Benzolen oder kondensierten aromatischen Ringsystemen mit Carbylsulfat in Gegenwart von Friedel-Crafts-Katalysatoren nicht, wie an sich vorstellbar, zu Verbindungen der Formel (I) gemäß dem Reaktionsschema

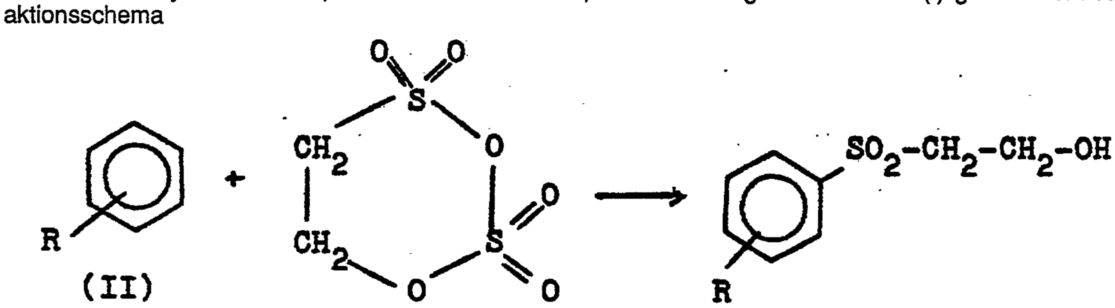

(II) (VII) (I)

führt, sondern zu Diarylethanderivaten.

Überraschenderweise wurde nun gefunden, daß man Aryloxethylsulfone der allgemeinen Formel (1)
$Ar-SO_2-CH_2-CH_2-OH$ (1),
in welcher Ar einen Phenyl- oder Naphthylrest bedeutet, in vorteilhafter Weise nach einem Einstufenverfahren herstellen kann, indem man einen Aromaten der allgemeinen Formel (2)
Ar-H (2),
in welcher Ar die vorstehend genannten Bedeutungen hat, mit Carbylsulfat in Gegenwart von Bortrichlorid, ggfs. in einem gegenüber den Reaktionskomponenten inerten organischen Lösemittel, bei Temperaturen von 30 bis 100°C, vorzugsweise 40 bis 60°C, umsetzt, dann durch Zusatz von Wasser oder Methanol das Bortrichlorid zersetzt und das gebildete Aryloxethylsulfon der Formel (1) isoliert.

Es ist zweckmäßig, Aromat (Benzol oder Naphthalin) und Carbylsulfat im Molverhältnis 1:1 einzusetzen, jedoch kann das Molverhältnis von 1:2 bis 5:1 variiert werden.

Das Bortrichlorid ist, bezogen auf das Carbylsulfat, mindestens im Molverhältnis 1:1 anzuwenden; es kann jedoch auch im molaren Überschuß bis 2:1 eingesetzt werden, wodurch aber nur eine mäßige Ausbeutesteigerung erzielt werden kann.

Als gegenüber den Reaktionskomponenten Carbylsulfat und Bortrichlorid inerte organische Lösemittel kommen aromatische Kohlenwasserstoffe, wie Benzol (wobei dieses bei der Umsetzung von Naphthalin, um den Erhalt von Mischprodukten zu vermeiden, ausscheidet), aliphatische oder cycloaliphatische Kohlenwasserstoffe, wie Octan, dessen Isomere oder Cyclohexan, ferner halogenierte, vorzugsweise chlorierte aromatische Kohlenwasserstoffe, wie Chlorbenzol oder Dichlorbenzol, halogenierte, vorzugsweise chlorierte aliphatische Kohlenwasserstoffe, wie Dichlormethan, Dichlorethan oder Propylchlorid, in Betracht.

Während bei der Verwendung von Benzol als Ausgangsverbindung der Zusatz eines inerten organischen Lösemittels nicht zwingend ist, ist bei der Verwendung von Naphthalin als Ausgangsverbindung ein solcher Zusatz erforderlich.

Zur Isolierung der gesuchten Endprodukte durch Extraktion eignen sich beispielsweise Alkyl$C_1$-$C_3$ carbonsäure-alkyl$C_1$-$C_4$-ester, wie Essigsäureethyl-, -propyl- oder -butylester, oder aromatische Kohlenwasserstoffe, wie Benzol, Toluol oder Xylole, oder halogenierte aromatische Kohlenwasserstoffe, wie Chlorbenzol oder Dichlorbenzol, oder Ketone wie Methyl-isobutyl-keton.

Es ist zweckmäßig, die Umsetzung so durchzuführen, daß man zu einer Suspension des Carbylsulfats im Aromaten eine Lösung des Bortrichlorids in einem inerten organischen Lösemittel zutropft bzw. das Bortrichlorid direkt in Gasform in die genannte Suspension einleitet und nach beendeter Reaktion den Katalysator mit Wasser oder Methanol zersetzt. Bei der Zersetzung des Bortrichlorids mit Wasser kann es zweckmäßig sein, ein Neutralisationsmittel, wie beispielsweise Natriumhydrogencarbonat, mit zuzusetzen. Die Reaktionszeit bewegt sich zwischen 30 Minuten und zwei Stunden.

Die Isolierung der Endprodukte, die durch Chromatographie vorgenommen werden kann, erfolgt vorzugsweise durch Extraktion.

Beispiel 1

Zu einer Suspension von 75,2 Teilen Carbylsulfat in 78 Teilen Benzol wird innerhalb von 20 Minuten bei 40-60°C eine Lösung von 47 Teilen Bortrichlorid in 312 Teilen Dichlorethan getropft. Dann wird 30 Minuten nachgerührt, mit 150 Teilen Wasser, 47 Teilen Natriumhydrogencarbonat sowie 100 Teilen Ethylacetat versetzt. Anschließend wird der ausgefallene Niederschlag abfiltriert und nach der Phasentrennung die wässrige Phase zweimal mit 100 Teilen Ethylacetat extrahiert. Nach Abdestillieren des Lösungsmittels erhält man 43,4 Teile Phenyloxethylsulfon, was einer Ausbeute von 58% der Theorie entspricht.

Beispiel 2

Man verfährt, wie in Beispiel 1 beschrieben, jedoch mit dem Unterschied, daß man statt mit Ethylacetat mit Methyl-isobutyl-keton extrahiert. Man erhält 42 Teile Phenyloxethylsulfon, was einer Ausbeute von 56% der Theorie entspricht.

Beispiel 3

Zu einer Mischung aus 12,8 Teilen Naphthalin und 37,6 Teilen Carbylsulfat wird innerhalb von 15 Minuten eine Lösung von 47 Teilen Bortrichlorid in 312 Teilen Dichlorethan getropft. Es wird 1 Stunde auf 30-45°C, dann 1/2 Stunde unter Rückfluß erhitzt. Anschließend wird mit 100 Teilen Methanol versetzt, weiterhin 30 Minuten unter Rückfluß erhitzt und das Lösungsmittel abdestilliert. Aus dem Sumpf erhält man nach Chromatographie an Kieselgel in Chlorbenzol/Dimethylformamid (2:1) 13 Teile 1-Naphthyloxethylsulfon, was einer Ausbeute von 55% der Theorie entspricht.

Beispiel 4

Man verfährt, wie in Beispiel 3 beschrieben, jedoch unter Zusatz von 100 Teilen Cyclohexan als Lösungsmittel für Naphthalin. Man erhält 12,5 Teile 1-Naphthyloxethylsulfon, was einer Ausbeute von 53% der Theorie entspricht.

**Patentansprüche**

1. Verfahren zur Herstellung von Aryloxethylsulfonen der allgemeinen Formel (I)
$Ar\text{-}SO_2\text{-}CH_2\text{-}CH_2\text{-}OH$, (1),
in welcher Ar einen Phenyl- oder Naphthylrest bedeutet, dadurch gekennzeichnet, daß man einen Aromaten der Formel (2)
Ar-H, (2),
in welcher Ar die vorstehend genannten Bedeutungen hat, mit Carbylsulfat in Gegenwart von Bortrichlorid, ggfs. in einem gegenüber den Reaktionskomponenten inerten organischen Lösemittel, bei Temperaturen von 30 bis 100°C umsetzt, dann durch Zusatz von Wasser oder Methanol das Bortrichlorid zersetzt und das gebildete Aryloxethylsulfon isoliert.

2. Verfahren nach Anspruch 1, dadurch gekennzeichnet, daß man die Reaktion bei Temperaturen von 40 bis 60°C vornimmt.

3. Verfahren nach Anspruch 1, dadurch gekennzeichnet, daß man die Reaktion in Benzol, Octan, Cyclohexan, Chlorbenzol, Dichlorbenzol, Dichlormethan, Dichlorethan oder Propylchlorid als inertem organischen Lösemittel durchgeführt.

4. Verfahren nach Anspruch 1, dadurch gekennzeichnet, daß man das Reaktionsprodukt isoliert durch Extraktion mit Essigsäure-ethyl-, propyl- oder butylester, Benzol, Toluol, Xylolen, Chlorbenzol, Dichlorbenzol oder Methyl-isobutyl-keton.

**Claims**

1. A process for preparing aryl hydroxyethyl sulfones of the formula (1)
$Ar\text{-}SO_2\text{-}CH_2\text{-}CH_2\text{-}OH$, (1),
in which Ar denotes a phenyl or naphthyl radical, which comprises reacting an aromatic of the formula (2)
Ar-H, (2),
in which Ar has the above mentioned meanings, with carbyl sulfate in the presence of boron trichloride at temperatures of 30 to 100°C, in the presence or absence of an organic solvent which is inert toward the reactants, then adding water or methanol to decompose the boron trichloride, and isolating the resulting aryl hydroxyethyl sulfone.

2. The process as claimed in claim 1, wherein the reaction is carried out at temperatures of 40 to 60°C.

3. The process as claimed in claim 1, wherein the reaction is carried out in benzene, octane, cyclohexane, chlorobenzene, dichlorobenzene, dichloromethane, dichloroethane or propyl chloride as inert organic solvent.

4. The process as claimed in claim 1, wherein the reaction product is isolated by extracting with ethyl, propyl or butyl acetate, benzene, toluene, xylenes, chlorobenzene, dichlorobenzene or methyl isobutyl ketone.

**Revendications**

1. Procédé de préparation d'aryl-hydroxy-éthylsulfones de formule générale 1 ci-dessous:
$Ar\text{-}SO_2\text{-}CH_2\text{-}CH_2\text{-}OH$, (1),
Ar désignant le radical phényle ou naphtyle, procédé caractérisé en ce que l'on fait réagir une matière aromatique de formule 2:
Ar-H, (2),
avec l'anhydride éthionique (sulfate de carbyle) en présence de trichlorure de bore, éventuellement dans un solvant organique inerte à l'égard des deux réactifs, à des températures de 30 à 100°C, puis on décompose le trichlorure de bore en ajoutant de l'eau ou du méthanol et on isole l'aryl-hydroxyéthylsulfone formée.

2. Procédé selon la revendication 1 caractérisé en ce que l'on effectue la réaction à des températures de 40 à 60°C.

3. Procédé selon la revendication 1 caractérisé en ce que l'on effectue la réaction dans du benzène, de l'octane, du cyclohexane, du chlorobenzène, du dichlorobenzène, du dichlorométhane, du dichloroéthane ou du chloropropane comme solvant inerte.

4. Procédé selon la revendication 1 caractérisé en ce que l'on isole le produit formé par extraction avec de l'acétate d'éthyle, de propyle ou de butyle, du benzène, du toluène, un xylène, du chlorobenzène, du dichlorobenzène ou de la méthylisobutylcétone.